# EUROPEAN PATENT SPECIFICATION

(11) **EP 4 039 298 B1**
(45) Date of publication and mention of the grant of the patent: **24.04.2024**
(21) Application number: 21305154.3
(22) Date of filing: 04.02.2021
(51) Int. Cl.: A61M 11/00, B05B 11/00, B05B 1/32, B05B 11/10, B05B 15/52

(54) **AUTOMATIC OSCILLATING SHUTTER DEVICE**
AUTOMATISCHE OSZILLIERENDE VERSCHLUSSVORRICHTUNG
DISPOSITIF OBTURATEUR À OSCILLATION AUTOMATIQUE

(43) Date of publication of application: 10.08.2022
(73) Proprietor: Eveon, 38330 Montbonnot-Saint-Martin (FR)
(72) Inventor: RABOT, Corentin, 38330 Montbonnot-Saint-Martin (FR); OUDOIRE, Patrick, 38330 Montbonnot-Saint-Martin (FR)
(74) Representative: Icosa

(56) References cited:
- WO-A2-2012/047887
- FR-A1- 2 796 921
- US-B2- 8 678 244

## Description

### FIELD OF INVENTION

The present invention relates to safe fluid distribution obturation.

### BACKGROUND OF INVENTION

Portable drug delivery devices have to take the preservation of the "cleanliness" and "integrity" of the device over time and the non-contamination of fluid circulating or stagnant within the device into consideration. In this regard, the administration port should be accessible as little as possible for this.

In the context of an injection device, the preservation of the microbiological quality of the product to be administered, the maintenance of the sterility of the needle but also its safe withdrawal are key features guaranteeing patient safety.

In the context of a spray or nebulization device, the risk of clogging of the administration orifice and alteration of the means of administration is high.

Finally, in the context of a reusable or semi-reusable device, it is preferable to be able to close the administration orifice directly after use to reduce these risks of clogging between two uses, which is harmful to the administration elements but also to the electronic or reusable parts.

More specifically, in spray drug delivery systems, one of the most complicated technical problems to solve is the risk of contamination of at least part of the fluid that is to be dispensed. This risk is accentuated by multiple use and the tiny residual amount systematically remaining at the administration port. This quantity of fluid remains in contact with the external environment through the dispensing orifice or the ejection nozzle, and can thus easily be subjected to drying and/or contamination, which leads to the following disadvantageous consequences. In the event of contamination, for example by bacteria, the contaminated fluid is distributed to the user during the next actuation, with represents an obvious danger to their health. In addition, the contamination could spread, in some cases, to the entire fluid reservoir with a consequent increased danger. In the case of drying, in addition to a very probable contamination, there is a risk of blocking the dispensing orifice of the dispensing member. This is very common especially in spray devices where the fluid passages are usually very small.

Sealing is thus essential and can be achieved by various means: internally or externally, automatic or manual.

In the case of a manual shutter, the following risks appear:
- a risk that the patient will not remove the shutter before initiating the administration of the product. In this case, there is a risk of not taking the dose by the patient as well as a risk of product leakage within the device.
- a risk that the patient does not close the shutter between two administrations of product or delays in closing the shutter after one administration. In this case, there is a risk of contamination of the administration outlet of the product and a risk of contamination of the remaining product to be administered.

In particular, documents EP031123 and US3336000 disclose closure systems in which the shutter is opened mechanically, independently of the pressure created by the product. These shutters, however form the end of the fluid flow portion and create a restriction in the free flow of fluid. There are even more conventional systems such as slides, caps, removable caps (see for example document WO2006013306). In particular, document US8678244 discloses a soap dispensing system comprising a fluid reservoir, a motor and a one-way valve in the form of a flap-type valve. This valve displays an open and a closed configuration. However, none of those examples solve the risk of the product drying out. These manual systems further entail the risk of misuse, loss, or breakage.

It is thus preferable to use an automatic shutter. However, those are complicated to couple to the delivery system and thus safely and effectively synchronize with the fluid distribution.

### SUMMARY

This invention thus relates to an automatic shutter device comprising:
- a fluid reservoir (12) configured to store some fluid,
- a fluid distribution system (14) connected to the fluid reservoir (12), the fluid distribution system (14) displaying a fluid distribution opening (16),
- a pump (18) comprising a pump cavity (20), the pump (18) being activable according to a rotative mode of operation, said rotative mode of operation comprising at least a suction phase during which the pump cavity (20) is configured to be filled with the fluid of the reservoir (12) and a discharge phase during which the pump cavity (20) is configured to be emptied,
- a motor (22) configured to activate the pump (18) in order to set the fluid into motion towards the fluid distribution opening (16), the motor (22) comprising at least one rotative element (26) connected to the pump (18),
- a shutter (24) configured to close the fluid distribution opening (16),
   **wherein**
- the shutter (24) displays at least two positions:
   o a shutting position in which the fluid distribution opening (16) is shut by the shutter (24),
   o an open position in which the fluid distribution opening (16) is free,
**characterized in that**
- the automatic shutter device (10) further comprises a movement conversion element (32) connected to the rotative element (26) of the motor (22) and configured to cooperate with the shutter (24), and
- the shutter (24) is configured to be moved from its shutting position to its distribution position and from its distribution position to its shutting position by means of the movement conversion element (32), the movements of the shutter (24) thus being configured to be synchronized with the rotation of the rotative element (26) of the motor (22).

Thus, this solution achieves the above objective. In particular, it allows the obtaining of:
- a tight sealing of the device,
- a reliable opening when the device is actuated,
- an obturation/opening without interfering with administration,
- a limitation of user actions.

The device according to the invention may include one or more of the following characteristics, taken in isolation from one another or in combination with one another:
- the pump is an oscillorotative pump,
- the movement conversion element is a driving cam,
- the motor is a rotative motor and rotates in a first direction in order to liberate the fluid distribution opening, and in a second direction in order to reset the position of the movement conversion element with regards to the rotative element,
- the shutter is connected to a sliding track and is configured to be translated, by the movement conversion element, along the sliding track in order to free or shut the distribution opening,
- the automatic shutter device is partly disposable,
- the fluid distribution system and the shutter are disposable,
- the pump is also disposable,
- the shutter comprises at least one cleaning element configured to clean the surroundings of the fluid distribution opening,
- the device further comprises a housing, the shutter being a mobile part of the housing.

A further object of the present invention is an automatic shutting process implemented by means of the automatic shutting device according to any one of the here above features, **wherein** the automatic shutting process comprises, once the motor of the automatic shutter device is activated, the rotative element and the movement conversion element put into rotation, the combined movement of both elements leading to at least four phases:
- a first phase during which the pump is inactivated, the pump cavity being empty and the shutter being in its shutting position,
- a second phase during which the pump is activated in its suction phase and the movement of the movement conversion element moves the shutter from its shutting position to its open position,
- a third phase during which the pump is activated in its discharge phase while the shutter in its open position,
- a fourth phase during which the pump is inactivated, the pump cavity remaining empty while the shutter is set into motion towards its shutting position,
the process starting again at the first phase once the fourth phase has ended.

In the process according to the present invention, the fourth phase may be longer than the second phase, or the fourth phase may be shorter than the second phase. The shutter movement during the fourth phase may be instantaneous. The duration of each phase may be determined by the design of the movement conversion element.

### BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration. purely illustrative and non-limiting examples, with reference to the accompanying drawings.
**Figure 1a** is a perspective view of a first embodiment of the device according to the present invention,
**Figure 1b** is a perspective detailed view of the fluidic system of the device according to the first embodiment,
**Figure 2** is a perspective view of the shutter of the embodiment of figures 1a and 1b,
**Figure 3a** is a perspective view of a second embodiment of the device according to the present invention in an open configuration,
**Figure 3b** is a perspective view of the second embodiment of the device according to the present invention in a shut configuration,
**Figure 3c** is a partial perspective view of the embodiment of figures 3a, 3b,
**Figure 4a** is a perspective view of a third embodiment of the device according to the present invention in an open configuration,
**Figure 4b** is a perspective view of the third embodiment of the device according to the present invention in a shut configuration,
**Figure 5a** is a perspective view of a fourth embodiment of the device according to the present invention in an open configuration,
**Figure 5b** is a perspective view of the fourth embodiment of the device according to the present invention in a shut configuration,
**Figure 6** is a succession of perspective views of a fifth embodiment in order to illustrate the four phases of the process according to the present invention.
**Figure 7a** is an embodiment of a pump cam according to the invention.
**Figure 7b** is an embodiment of a movement conversion element.
**Figure 8** is a perspective view of the first embodiment of the device enclosed in a housing.
**Figures 9a** and **9b** are two schematic representations of the process according to the present invention.

### DETAILED DESCRIPTION

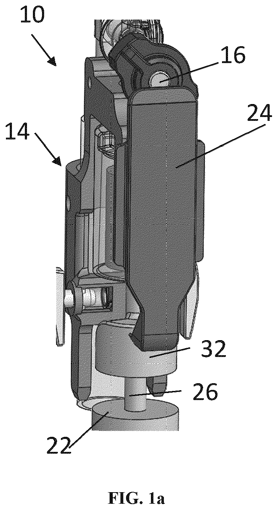

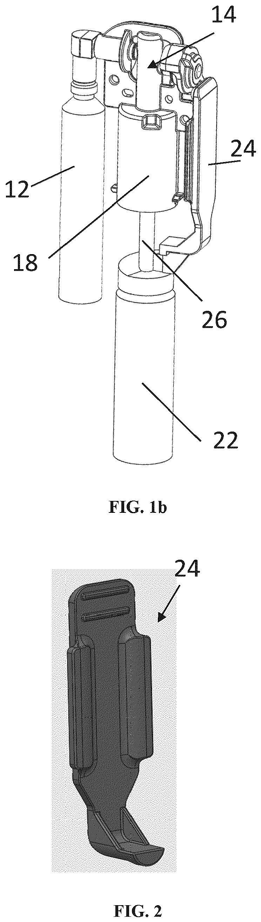

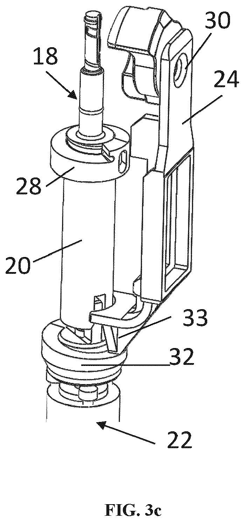

As can be seen on figure 1, the automatic shutter device 10 according to the present invention comprises:
- a fluid reservoir 12 (see figures 1b, 5a, 5b and 6) configured to store some fluid,
- a fluid distribution system 14 connected to the fluid reservoir 12, the fluid distribution system 14 displaying a fluid distribution opening 16,
- a pump 18 comprising a pump cavity 20 (see figure 3c),
- a motor 22 configured to activate the pump 18 in order to set the fluid from the reservoir 12 into motion towards the fluid distribution opening 16,
- a shutter 24 configured to close the fluid distribution opening 16.

The fluid distribution system 14 is secured, removably or not, to the pump 18 and may comprise a needle or a nozzle. It is put in fluidic communication with the fluid reservoir 12 by means of the pump 18.

According to the present invention, the pump 18 is activable according to a rotative mode of operation, said rotative mode of operation comprising at least a suction phase and a discharge phase depending on the rotation. During the suction phase, the pump cavity 20 is configured to be filled with the fluid of the reservoir 12, and during the discharge phase the pump cavity 20 is configured to be emptied, the fluid being pushed towards the fluid distribution opening 16. The pump 18 may be a rotary type of volumetric pump, or a rotary-oscillating (oscillorotative) type of pump, or a piston type of pump. In each case, the pump 18 does display a rotation induced suction phase and discharge phase. In the embodiments depicted on figures 1a, 3a, 3b, 4a, 4b and 5a, 5b, the pump 18 extends along an oscillation axis A.

The motor 22 is a classical rotative motor. The motor 22 thus comprises at least one rotative element 26 connected to the pump 18. The rotative element 26 may be directly or indirectly connected to the pump 18, for example by means of an intermediate connection element. In the different embodiments represented on the figures, the rotative element 26 is a rotative rod which rotates around the oscillation axis A of the pump 18. Said rod is further connected to a pump cam 28 (see figure 3c) which aims at transforming the rotative movement of the rotative element 26 of the motor 22 into an oscillating movement of the pump 18. This oscillating movement of the pump 18 determines the duration of the suction phase and discharge phase of said pump 18. Thus, the shape of the pump cam 28 is directly correlated with the duration of the suction and discharge phases. If the pump cam 28 displays smooth slopes, the phase(s) display long durations, if the pump cam 28 displays sharp edges, the phase(s) display short duration(s), possibly being instantaneous. The pump cam 28 may or may not form one single piece with the pump 18.

The shutter 24 can take different forms depending on the embodiment of the device 10. Regardless of the embodiment, the shutter 24 is a rigid piece secured to the fluid distribution system 14 in a movable way. It thus displays at least two positions:
o a shutting position in which the fluid distribution opening 16 is shut by the shutter 24,
o an open position in which the fluid distribution opening 16 is free.

The shutting position prevents any fluid to flow through the distribution opening 16, in any direction. On the opposite, the open position enables fluid to cross the distribution opening 16.

Regarding the embodiments depicted in figures 1a, 1b, 2, 3a, 3b and 3c, the shutter 24 is an elongated piece of hard fabric, for example plastics. In some alternative embodiments, it might be an elongated piece of flexible fabric. In those embodiments, the shutter 24 is secured to the fluid distribution system 14 in order to be able to move up and down along the pump oscillation axis A. More precisely, in those embodiments, the shutter 24 is connected to a sliding track secured to the fluid distribution system 14 along the oscillation axis A, and is able to translate along said sliding track in order to free or shut the distribution opening 16.

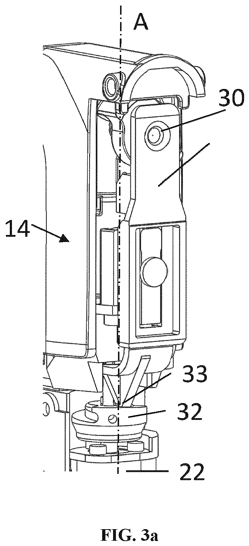

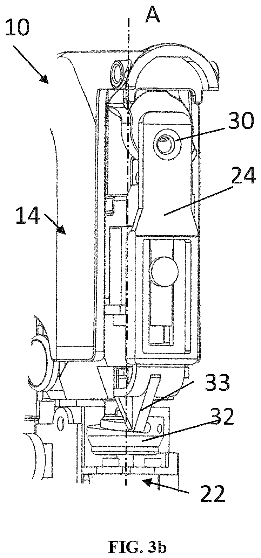

In the embodiment of figure 1, the shutter 24 is a plain piece of fabric. Its shutting position is the axially highest position along the oscillation axis A (see figure 1), in which it covers the distribution opening 16. In this embodiment, the open position of the shutter 24 is the lowest position along the oscillation axis A, in which the higher edge of the shutter is below the fluid distribution opening 16 regarding the oscillation axis A. In the embodiment of figures 3a, 3b and 3c, the shutter 24 displays an aperture 30 presenting sensibly the same diameter than the fluid distribution opening 16. In this embodiment, the shutting position is the axially lowest position along the oscillation axis A, in which the plain part of the shutter 24 covers the distribution opening 16 (see figure 3b). Its open position is the axially highest position along the oscillation axis A, in which the aperture 30 is aligned with the fluid distribution opening 16 (see figure 3a).

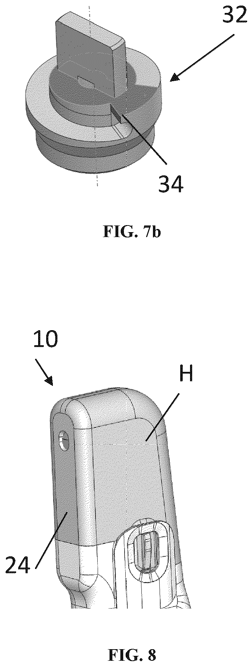

In some embodiments (see figure 8), the device 10 further comprises a housing and the shutter 24 is a mobile part of this housing.

As can be seen on figure 2, it is possible to add some further functional elements on the shutter 24, as for example such as seals and/or cleaning elements 31 making it possible to clean (for example by scraping or wiping) the surroundings of the fluid distribution opening 16 or to absorb the remaining fluid residues.

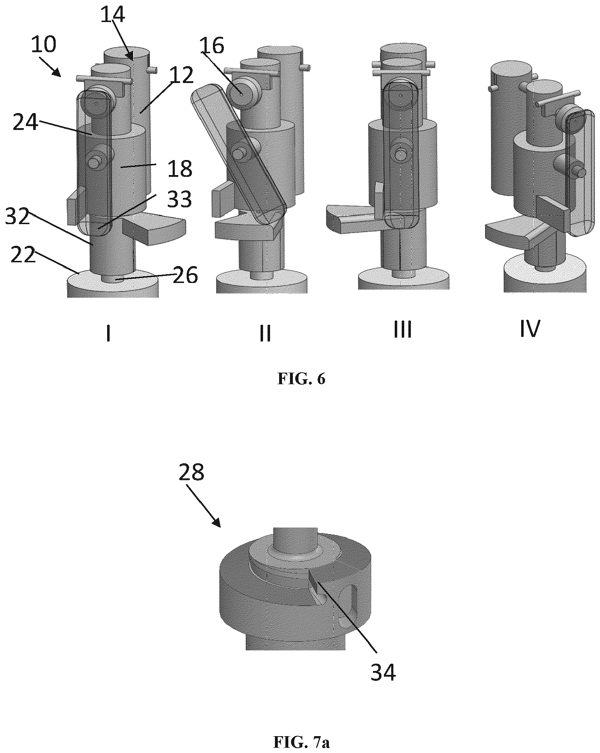

Regarding the embodiment depicted on figure 6, the shutter 24 is also an elongated piece, however the opening and shutting of the distribution opening 16 happens by means of a lateral oscillating movement in front of the fluid distribution system 14. This embodiment is advantageous in the embodiments displaying the shutter 24 on the external part of the device 10. However, this embodiment implies a lateral opening which requests a wider span of the device 10 in its active stage.

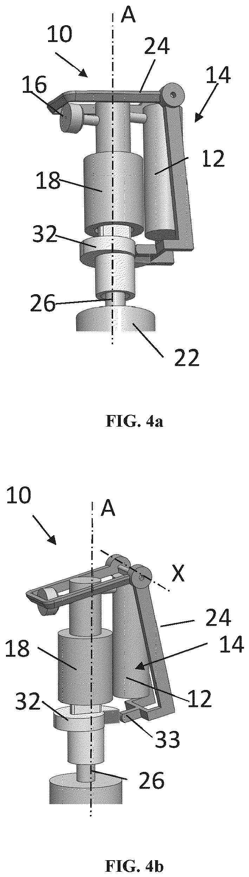

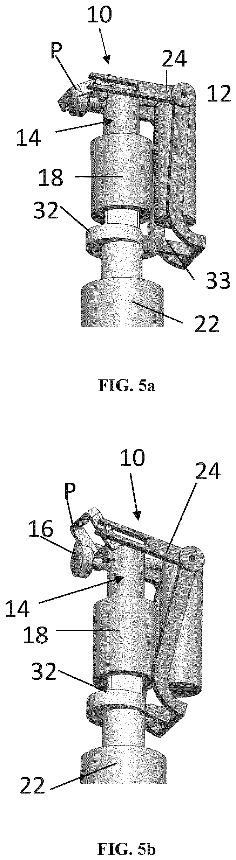

Regarding the embodiments depicted on figure 4a, 4b, 5a and 5b, the shutter 24 is an oscillating flange of hard fabric, for example plastics. In those embodiments, the shutter 24 is secured to the fluid distribution system 14 by means of a secondary oscillation axis X sensibly orthogonal to the oscillation axis A, in order to be able to move back and forth around the fluid distribution system 14. In the embodiment of figures 5a and 5b, the flange further comprises a sliding plug P configured to plug the distribution opening 16 when the shutter 24 is in its shutting position. The plug P enables to improve the watertightness of the shutter 24.

Regardless of the embodiment, the shutter 24 is configured to be moved from its shutting position to its distribution position and from its distribution position to its shutting position by means of a movement conversion element 32 connected to the rotative element 26 of the motor 22. This movement conversion element 32 is for example a driving cam (see figures 1 and 3a) connected to the rotative element 26 of the motor 22. In the same way as the pump cam 28 for the pump 18, the movement conversion element 32 converts the rotative movement of the rotative element 26 of the motor 22 into an oscillating movement of the shutter 24. The movements of the shutter 24 is thus synchronized with the rotation of the rotative element 26 of the motor 22 and thus, needs to be synchronized with the rotation and design of the pump cam 28 in order to be synchronized with the oscillating movement of the pump 18.

In some embodiments, the shutter 24 cooperates both with the movement conversion element 32 and with the pump cam 28.

As already stated, in the embodiment of figures 1a and 2, the movement conversion element 32 is a driving cam, and more precisely, a rotary drum cam (also called bell cam in the prior art). It is nevertheless possible to consider other mechanical means of driving and transforming movement. For example, a groove cam which allows direct axial translation or a disc cam which allows radial translation to be converted into axial translation.

In order to cooperate with the movement conversion element 32, the shutter 24 comprises an abutment element 33 configured to cooperate by sliding, with the movement conversion element 32. In the embodiments depicted on figures 4a, 4b, 5a and 5b, the movement conversion element 32 is designed to cooperate with a particular abutment element 33 being an angle transmission, thus enabling the movement of a "short" rotation similar to a quasi-translation of the shutter over a small distance (approximatively 3mm). This angle transmission can be coupled to a return spring in order to facilitate the shutter 24 reset.

In order to improve safety and hygiene, the device 10 according to present invention may be partly disposable. More precisely, in a hygienic perspective, every piece of the device 10 in contact with the fluid to be distributed might be disposable, in particular the fluid distribution system 14 and the shutter 24 are disposable. In some embodiments, the pump 18 is also disposable. This way, only the electromechanical elements are designed to be reusable: more precisely, in a partly disposable perspective, solely the motor 22 and the movement conversion element 32 are reusable.

Over time, after a certain number of turns, there is a risk of a slight angular shift of the movement conversion element 32 with regards to the rotative element 26 of the motor 22 at each new turn. The accumulation of those slight angular shifts could, in the end, lead to a significant angular shift, this resulting in:
- the device 10 wrongly administering a dose of fluid instead of remaining in the "shutter open, pump loaded" step (see further below) if the "run" of the movement conversion element 32 is exceeded,
- failure to administer the required fluid dose if the "rate" of the movement conversion element 32 is not exceeded.

To prevent this, a homing step can be added: the rotative element 26 of the motor 22 rotates in a first direction in order to liberate the fluid distribution opening 16, and after fluid distribution, the motor 22 rotates in a second direction in order to reset the position of the movement conversion element 32 with regards to the rotative element 26 and thus, to the pump cam 28. This position defines the 0° position and thus allows the adjustment of the 0° position after each revolution. More precisely, each one of the movement conversion element 32 and the pump cam 28 displays at least one sharp edge/step 34 (see figures 7a and 7b). Depending on the embodiments, this homing step can be done either on the pump cam 28 or on movement conversion element 32. The homing step takes place when the sharp edge/step 34 of either the pump cam 28 or the movement conversion element 32 has just been passed: the motor 22 goes back so that the sharp edge/step of the pump cam 28 or of the movement conversion element 32 comes into abutment with the abutment element 33. In the embodiments in which the pump cam 28 is designed to cooperate (in addition to the movement conversion element 32) with the shutter 24, it is advantageous to use the pump cam 28 for the homing step because the pump cam 28 is less fragile than the movement conversion element 32 (risk of breakage, of deformation).

In a further embodiment (not shown), it is possible to perform the homing step without any sharp edge/step on the pump cam 28 or the movement conversion element 32. This embodiment requests an electronic control of the motor, and implemented sensors to perform the homing step.

The device 10 according to the present invention, enables the implementation of an automatic shutting process of a fluid distribution device illustrated on figure 6. Regarding the present invention, the automatic shutting process comprises, once the motor 22 of the automatic shutter device 10 is activated and the rotative element 26 and the movement conversion element 32 are put into rotation, as the combined movement of both elements 26, 32 do lead to at least four phases:
- a first phase I during which the pump 18 is inactivated, the pump cavity 20 being empty and the shutter 24 being in its shutting position,
- a second phase II during which the pump 18 is activated in its suction phase and the movement of the movement conversion element 32 moves the shutter 24 from its shutting position to its open position, the pump 18 activation and the opening of the shutter 24 happening either simultaneously or sequentially,

- a third phase III during which the pump 18 is activated in its discharge phase while the shutter 24 remains in its open position,
- a fourth phase IV during which the pump 18 is inactivated, the pump cavity 20 remaining empty while the shutter 24 is set into motion towards its shutting position.

If the process includes a homing step, it takes place between the third phase III and the fourth phase IV. This step may happen as follows (or in the opposite directions):
- loading of the pump 18 / opening of the shutter 24 (motor 22 rotating clockwise),
- fluid administration / open shutter 24 (motor 22 rotating clockwise),
- homing on the pump cam 28 (motor 22 rotating counter clockwise),
- closing of the shutter 24 (motor 22 rotating clockwise).

The process can start again at the first phase I once the fourth IV phase has ended, as long there is fluid to be distributed in the reservoir 12.

In order to allow complete administration of the fluid, the movement of the shutter 24 to its shutting position must be offset (posteriorly) with respect to the discharge phase of the pump 18. Thus, the shutter 24 must shut the distribution opening 16 after the end of the distribution (for example a spray or an injection sequence).

Generally speaking, the shutter opening and shutting speeds are dependent on the rotational speed of the rotative element 26 of the motor 22. These speeds are therefore correlated with the suction/discharge speed of the pump 18. However, these speeds may vary depending on the pump cam 28 and movement conversion element 32 profiles:
- the closing speed can be very fast if movement conversion element 32 with a sharp step, whether the pump 18 delivery is sudden (pump cam 28 with sharp slope, or step) or progressive (pump cam 28 with smooth slope),
- the shutting speed can be slower if the movement conversion element 32 displays a smooth slope instead of a step,
- the opening speed of the shutter 24 can almost be identical to the suction speed of the pump 18 (similar slopes on the pump cam 28 and the movement conversion element 32, angles of the opening and suction phases almost identical) but the opening speed could be faster if the opening began at a higher angle than the start of pump suction. The "speed" of opening is limited by the "steepness" of the slope of the movement conversion element 32.

In order for the process to be effective, it needs at least a complete shutter 24 opening/shutting - pump 18 loading/unloading sequence on 360 ° of rotation of the rotative element 26 of the motor 22 to which the pump cam 28 and the movement conversion element 32 are connected. In some embodiments, there might be several shutter 24 open/closed - pump 18 loading/unloading sequences over 360 ° but there are preferably the same number of shutter 24 open/shutting sequences as of pump 18 loading/unloading.

In some embodiments, the fourth phase IV is longer than the second phase II, leading to a quick opening and a smoother shutting of the distribution opening 16. In an alternative embodiment, the fourth phase IV is shorter than the second phase II, leading to a smooth opening and a quick shutting of the distribution opening 16. The shutter movement during the fourth phase IV could be instantaneous.

Generally speaking, the same way that the cam pump 28 design determines the duration of the suction and discharge phases of the pump 18, the duration of each phase I, II, III, IV of the process is determined by the design of the movement conversion element 32.

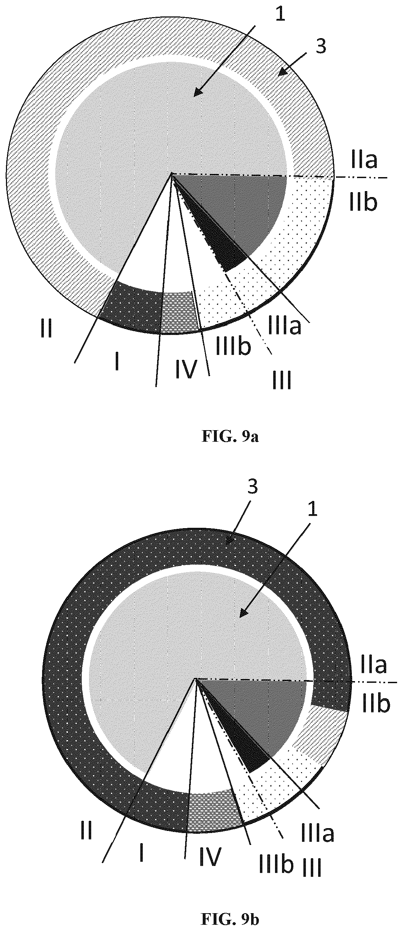

Figures 9a and 9b illustrates two particular embodiments of the process according to the present invention.

In both figures, after the first phase I (inactivated pump 18 and shutter 24 in shutting position), comes the second phase II which breaks down into a first subphase IIa during which the pump 18 is charging and a second subphase IIb during which the pump cavity 20 is full. Then comes the third phase III which also breaks down into a first subphases IIIa during which the fluid distribution takes place, meaning the fluid is moved through the distribution opening 16, and a second subphase IIIb during which the pump cavity 20 is completely empty. The fourth phase IV corresponds to the shut state of the device 10.

As can further be seen on figures 9a and 9b, to each state of the pump 18, there is a corresponding shutter 24 state. During the first phase I, the shutter 24 is in its shutting position. During the second phase II, the latest at the end of phase lib, the shutter 24 must have switched to its open position. During the third phase III, it is necessary that the shutter 24 remains in its open position over the complete duration of the third phase III. During the fourth phase IV, the shutter 24 changes from its open position to its shutting position and the device 10 comes back to the first phase I.

The two examples of figures 9a and 9b thus illustrates two embodiments with different durations of the different phases I, II, III, IV. The present invention is about an automated shutting device 10 configured to avoid two of the here-above introduced key issues:
- reducing the number of user actions upon the device 10: a manual shutter necessitates opening then closing,
- reducing the risk of misuse: forgetting to open/close before/after use:
   ∘ in the case of forgetting to open the shutter, the risk is to induce product leakage into the distribution device, thus causing its premature deterioration,
   o in the case of forgetting to close the shutter, the risk is to exposes the device to dust or other contaminants being deposited in the distribution opening and thus contaminating the product to be administered remaining inside the fluid reservoir.

## Claims

1. Automatic shutter device (10) comprising:
- a fluid reservoir (12) configured to store some fluid,
- a fluid distribution system (14) connected to the fluid reservoir (12), the fluid distribution system (14) displaying a fluid distribution opening (16),
- a pump (18) comprising a pump cavity (20), the pump (18) being activable according to a rotative mode of operation, said rotative mode of operation comprising at least a suction phase during which the pump cavity (20) is configured to be filled with the fluid of the reservoir (12) and a discharge phase during which the pump cavity (20) is configured to be emptied,
- a motor (22) configured to activate the pump (18) in order to set the fluid into motion towards the fluid distribution opening (16), the motor (22) comprising at least one rotative element (26) connected to the pump (18),
- a shutter (24) configured to close the fluid distribution opening (16),
**wherein**
- the shutter (24) displays at least two positions:
o a shutting position in which the fluid distribution opening (16) is shut by the shutter (24),
o an open position in which the fluid distribution opening (16) is free,
**characterized in that**
- the automatic shutter device (10) further comprises a movement conversion element (32) connected to the rotative element (26) of the motor (22) and configured to cooperate with the shutter (24), and
- the shutter (24) is configured to be moved from its shutting position to its distribution position and from its distribution position to its shutting position by means of the movement conversion element (32), the movements of the shutter (24) thus being configured to be synchronized with the rotation of the rotative element (26) of the motor (22).

2. Automatic shutter device (10) according to claim 1, **wherein** the pump (18) is an oscillorotative pump.

3. Automatic shutter device (10) according to any one of the preceding claims, **wherein** the movement conversion element (32) is a driving cam.

4. Automatic shutter device (10) according to the preceding claim, **wherein** the motor (22) is a rotative motor and rotates in a first direction in order to liberate the fluid distribution opening (16), and in a second direction in order to reset the position of the movement conversion element (32) with regards to the rotative element (26).

5. Automatic shutter device (10) according to any one of the preceding claims, **wherein** the shutter (24) is connected to a sliding track and is configured to be translated, by the movement conversion element (32), along the sliding track in order to free or shut the distribution opening (16).

6. Automatic shutter device (10) according to any one of the preceding claims, **wherein** the automatic shutter device (10) is partly disposable.

7. Automatic shutter device (10) according to the preceding claim, **wherein** the fluid distribution system (14) and the shutter (24) are disposable.

8. Automatic shutter device (10) according to the preceding claim, **wherein** the pump (18) is also disposable.

9. Automatic shutter device (10) according to any one of the preceding clams, **wherein** the shutter (24) comprises at least one cleaning element (31) configured to clean the surroundings of the fluid distribution opening (16).

10. Automatic shutter device (10) according to any one of the preceding claims, **wherein** the device further comprises a housing (H), the shutter (24) being a mobile part of the housing (H).

11. Automatic shutting process implemented by means of the automatic shutting device (10) according to any one of the preceding claims,
**wherein** the automatic shutting process comprises, once the motor (22) of the automatic shutter device (10) is activated, the rotative element (26) and the movement conversion element (32) put into rotation, the combined movement of both elements (26, 32) leading to at least four phases:
- a first phase (I) during which the pump (18) is inactivated, the pump cavity (20) being empty and the shutter (24) being in its shutting position,
- a second phase (II) during which the pump (18) is activated in its suction phase and the movement of the movement conversion element (32) moves the shutter (24) from its shutting position to its open position,
- a third phase (III) during which the pump (18) is activated in its discharge phase while the shutter (24) in its open position,
- a fourth phase (IV) during which the pump (18) is inactivated, the pump cavity (20) remaining empty while the shutter (24) is set into motion towards its shutting position,
the process starting again at the first phase (I) once the fourth phase (IV) has ended.

12. Automatic shutting process according to the preceding claim, **wherein** the fourth phase (IV) is longer than the second phase (II).

13. Automatic shutting process according to claim **11, wherein** the fourth phase (IV) is shorter than the second phase (II).

14. Automatic shutting process according to any one of claims **11** to **13, wherein** the shutter movement during the fourth phase (IV) is instantaneous.

15. Automatic shutting process according to any one of claims **11** to **14, wherein** the duration of each phase (I, II, III, IV) is determined by the design of the movement conversion element (32).

## Patentansprüche

1. Automatische Verschlussvorrichtung (10), umfassend:
- ein Fluidreservoir (12), das dazu konfiguriert ist, Fluid zu speichern,
- ein Fluidverteilungssystem (14), das mit dem Fluidreservoir (12) verbunden ist, wobei das Fluidverteilungssystem (14) eine Fluidverteilungsöffnung (16) aufweist,
- eine Pumpe (18), die einen Pumpenhohlraum (20) umfasst, wobei die Pumpe (18) gemäß einem Drehbetriebsmodus aktivierbar ist, wobei der Drehbetriebsmodus mindestens eine Saugphase, während der der Pumpenhohlraum (20) dazu konfiguriert ist, mit dem Fluid des Reservoirs (12) gefüllt zu werden, und eine Entladephase umfasst, während der der Pumpenhohlraum (20) dazu konfiguriert ist, entleert zu werden,
- einen Motor (22), der dazu konfiguriert ist, die Pumpe (18) zu aktivieren, um das Fluid in Richtung der Fluidverteilungsöffnung (16) in Bewegung zu versetzen, wobei der Motor (22) mindestens ein sich drehendes Element (26) umfasst, das mit der Pumpe (18) verbunden ist,
- einen Verschluss (24), der dazu konfiguriert ist, die Fluidverteilungsöffnung (16) zu verschließen,
wobei
- der Verschluss (24) mindestens zwei Positionen aufweist:
o eine Verschlussposition, in der die Fluidverteilungsöffnung (16) durch den Verschluss (24) verschlossen ist,
o eine offene Position, in der die Fluidverteilungsöffnung (16) frei ist,
**dadurch gekennzeichnet, dass**
- die automatische Verschlussvorrichtung (10) ferner ein Bewegungsumwandlungselement (32) umfasst, das mit dem sich drehenden Element (26) des Motors (22) verbunden und dazu konfiguriert ist, mit dem Verschluss (24) zusammenzuwirken, und
- der Verschluss (24) dazu konfiguriert ist, mittels des Bewegungsumwandlungselements (32) von seiner Verschlussposition in seine Verteilungsposition und von seiner Verteilungsposition in seine Verschlussposition bewegt zu werden, wobei die Bewegungen des Verschlusses (24) somit dazu konfiguriert sind, mit der Drehung des sich drehenden Elements (26) des Motors (22) synchronisiert zu werden.

2. Automatische Verschlussvorrichtung (10) nach Anspruch 1, wobei die Pumpe (18) eine oszillatorische Pumpe ist.

3. Automatische Verschlussvorrichtung (10) nach einem der vorhergehenden Ansprüche, wobei das Bewegungsumwandlungselement (32) ein Antriebsstrang ist.

4. Automatische Verschlussvorrichtung (10) nach dem vorhergehenden Anspruch, wobei der Motor (22) ein Drehmotor ist und sich in einer ersten Richtung dreht, um die Fluidverteilungsöffnung (16) freizugeben, und in einer zweiten Richtung, um die Position des Bewegungsumwandlungselements (32) in Bezug auf das Drehelement (26) zurückzusetzen.

5. Automatische Verschlussvorrichtung (10) nach einem der vorhergehenden Ansprüche, wobei der Verschluss (24) mit einer Gleitschiene verbunden und dazu konfiguriert ist, durch das Bewegungsumwandlungselement (32) entlang der Gleitschiene verschoben zu werden, um die Verteileröffnung (16) freizugeben oder zu verschließen.

6. Automatische Verschlussvorrichtung (10) nach einem der vorhergehenden Ansprüche, wobei die automatische Verschlussvorrichtung (10) teilweise zur Einmalanwendung ist.

7. Automatische Verschlussvorrichtung (10) nach dem vorhergehenden Anspruch, wobei das Fluidverteilungssystem (14) und der Verschluss (24) zur Einmalanwendung sind.

8. Automatische Verschlussvorrichtung (10) nach dem vorhergehenden Anspruch, wobei die Pumpe (18) ebenfalls zur Einmalanwendung ist.

9. Automatische Verschlussvorrichtung (10) nach einem der vorhergehenden Ansprüche, wobei der Verschluss (24) mindestens ein Reinigungselement (31) umfasst, das dazu konfiguriert ist, die Umgebung der Fluidverteilungsöffnung (16) zu reinigen.

10. Automatische Verschlussvorrichtung (10) nach einem der vorhergehenden Ansprüche, wobei die Vorrichtung ferner ein Gehäuse (H) umfasst, wobei der Verschluss (24) ein beweglicher Teil des Gehäuses (H) ist.

11. Automatisches Schließverfahren, das mittels der automatischen Verschlussvorrichtung (10) nach einem der vorhergehenden Ansprüche implementiert wird,
wobei das automatische Schließverfahren das sich drehende Element (26) und das in Drehung versetzte Bewegungsumwandlungselement (32) umfasst, sobald der Motor (22) der automatischen Verschlussvorrichtung (10) aktiviert wird, wobei die kombinierte Bewegung beider Elemente (26, 32) zu mindestens vier Phasen führt:
- einer ersten Phase (I), während der die Pumpe (18) deaktiviert ist, wobei der Pumpenhohlraum (20) leer ist und sich der Verschluss (24) in seiner Schließposition befindet,
- einer zweiten Phase (II), während der die Pumpe (18) in ihrer Saugphase aktiviert wird und die Bewegung des Bewegungsumwandlungselements (32) den Verschluss (24) von seiner Schließposition in seine offene Position bewegt,
- einer dritten Phase (III), während der die Pumpe (18) in ihrer Entladephase aktiviert wird, während sich der Verschluss (24) in seiner offenen Position befindet,
- einer vierten Phase (IV), während der die Pumpe (18) deaktiviert ist, wobei der Pumpenhohlraum (20) leer bleibt, während der Verschluss (24) in Richtung seiner Schließposition in Bewegung versetzt wird,
wobei das Verfahren wieder mit der ersten Phase (I) beginnt, sobald die vierte Phase (IV) beendet ist.

12. Automatisches Schließverfahren nach dem vorhergehenden Anspruch, wobei die vierte Phase (IV) länger ist als die zweite Phase (II).

13. Automatisches Schließverfahren nach Anspruch 11, wobei die vierte Phase (IV) kürzer ist als die zweite Phase (II).

14. Automatisches Schließverfahren nach einem der Ansprüche 11 bis 13, wobei die Verschlussbewegung während der vierten Phase (IV) augenblicklich erfolgt.

15. Automatisches Schließverfahren nach einem der Ansprüche 11 bis 14, wobei die Dauer jeder Phase (I, II, III, IV) durch die Planung des Bewegungsumwandlungselements (32) bestimmt wird.

## Revendications

1. Dispositif d'obturation automatique (10) comprenant :
- un réservoir de fluide (12) configuré pour stocker du fluide,
- un système de distribution de fluide (14) relié au réservoir de fluide (12), le système de distribution de fluide (14) présentant une ouverture de distribution de fluide (16),
- une pompe (18) comprenant une cavité de pompe (20), la pompe (18) pouvant être activée selon un mode de fonctionnement rotatif, ledit mode de fonctionnement rotatif comprenant au moins une phase d'aspiration pendant laquelle la cavité de pompe (20) est configurée pour être remplie avec le fluide du réservoir (12) et une phase de refoulement pendant laquelle la cavité de pompe (20) est configurée pour être vidée,
- un moteur (22) configuré pour activer la pompe (18) afin de mettre le fluide en mouvement vers l'ouverture de distribution du fluide (16), le moteur (22) comprenant au moins un élément rotatif (26) connecté à la pompe (18),
- un obturateur (24) configuré pour fermer l'ouverture de distribution du fluide (16),
**dans lequel**
l'obturateur (24) présente au moins deux positions :
- une position fermée dans laquelle l'ouverture de distribution de fluide (16) est fermée par l'obturateur (24),
- une position ouverte dans laquelle l'ouverture de distribution du fluide (16) est libre,
**caractérisé en ce que**
- le dispositif d'obturation automatique (10) comprend en outre un élément de conversion de mouvement (32) relié à l'élément rotatif (26) du moteur (22) et configuré pour coopérer avec l'obturateur (24), et
- l'obturateur (24) est configuré pour être déplacé de sa position de fermeture à sa position de distribution et de sa position de distribution à sa position de fermeture au moyen de l'élément de conversion de mouvement (32), les mouvements de l'obturateur (24) étant ainsi configurés pour être synchronisés avec la rotation de l'élément rotatif (26) du moteur (22).

2. Dispositif d'obturation automatique (10) selon la revendication 1, **dans lequel** la pompe (18) est une pompe oscillo-rotative.

3. Dispositif d'obturation automatique (10) selon l'une quelconque des revendications précédentes, **dans lequel** l'élément de conversion de mouvement (32) est une came d'entraînement.

4. Dispositif d'obturation automatique (10) selon la revendication précédente, dans lequel le moteur (22) est un moteur rotatif et tourne dans un premier sens pour libérer l'ouverture de distribution du fluide (16), et dans un deuxième sens pour réinitialiser la position de l'élément de conversion de mouvement (32) par rapport à l'élément rotatif (26).

5. Dispositif d'obturation automatique (10) selon l'une quelconque des revendications précédentes, **dans lequel** l'obturateur (24) est relié à un rail de glissement et est configuré pour être translaté, par l'élément de conversion de mouvement (32), le long du rail de glissement afin de libérer ou de fermer l'ouverture de distribution (16).

6. Dispositif d'obturation automatique (10) selon l'une quelconque des revendications précédentes, **dans lequel** le dispositif d'obturation automatique (10) est partiellement jetable.

7. Dispositif d'obturation automatique (10) selon la revendication précédente, **dans lequel** le système de distribution de fluide (14) et l'obturateur (24) sont jetables.

8. Dispositif d'obturation automatique (10) selon la revendication précédente, **dans lequel** la pompe (18) est également jetable.

9. Dispositif d'obturation automatique (10) selon l'une quelconque des revendications précédentes, **dans lequel** l'obturateur (24) comprend au moins un élément de nettoyage (31) configuré pour nettoyer les environs de l'ouverture de distribution de fluide (16).

10. Dispositif d'obturation automatique (10) selon l'une quelconque des revendications précédentes, **dans lequel** le dispositif comprend en outre un boîtier (H), l'obturateur (24) étant une partie mobile du boîtier (H).

11. Procédé de fermeture automatique mis en oeuvre au moyen du dispositif de fermeture automatique (10) selon l'une quelconque des revendications précédentes,
**dans lequel** le procédé de fermeture automatique comprend, une fois que le moteur (22) du dispositif de fermeture automatique (10) est activé, la mise en rotation de l'élément rotatif (26) et de l'élément de conversion de mouvement (32), le mouvement combiné des deux éléments (26, 32) conduisant à au moins quatre phases :
- une première phase (I) pendant laquelle la pompe (18) est inactivée, la cavité de la pompe (20) étant vide et l'obturateur (24) étant dans sa position fermée,
- une deuxième phase (II) au cours de laquelle la pompe (18) est activée dans sa phase d'aspiration et le mouvement de l'élément de conversion de mouvement (32) fait passer l'obturateur (24) de sa position fermée à sa position d'ouverture,
- une troisième phase (III) au cours de laquelle la pompe (18) est activée dans sa phase de refoulement alors que l'obturateur (24) est en position ouverte,
- une quatrième phase (IV) pendant laquelle la pompe (18) est inactivée, la cavité de la pompe (20) restant vide tandis que l'obturateur (24) est mis en mouvement vers sa position fermée,
le procédé recommençant à la première phase (I) une fois que la quatrième phase (IV) est terminée.

12. Procédé de fermeture automatique selon la revendication précédente, **dans lequel** la quatrième phase (IV) est plus longue que la deuxième phase (II).

13. Procédé de fermeture automatique selon la revendication 11, **dans lequel** la quatrième phase (IV) est plus courte que la deuxième phase (II).

14. Procédé de fermeture automatique selon l'une quelconque des revendications 11 à 13, dans lequel le mouvement du volet pendant la quatrième phase (IV) est instantané.

15. Procédé de fermeture automatique selon l'une des revendications 11 à 14, **dans lequel** la durée de chaque phase (I, II, III, IV) est déterminée par la conception de l'élément de conversion de mouvement (32).
